Europäisches Patentamt

European Patent Office

Office européen des brevets

(11) Publication number: **0 343 703**
**A2**

(12) **EUROPEAN PATENT APPLICATION**

(21) Application number: 89201188.3

(51) Int. Cl.⁴: **A23L 1/304** , **A23L 2/26** ,
**A23L 1/236**

(22) Date of filing: **11.05.89**

(30) Priority: **26.05.88 US 198955**

(43) Date of publication of application:
**29.11.89 Bulletin 89/48**

(84) Designated Contracting States:
**AT BE CH DE ES FR GB GR IT LI LU NL SE**

(71) Applicant: THE PROCTER & GAMBLE
COMPANY
One Procter & Gamble Plaza
Cincinnati Ohio 45202(US)

(72) Inventor: **Heckert, David Clinton**
**3561 Kehr Road**
**Oxford, OH 45056(US)**
Inventor: **Mehansho, Haile**
**7 Highknoll Court**
**Fairfield, OH 45014(US)**
Inventor: **Nakel, Gunther Maria**
**R. R. 1, Box 836**
**Aurora, IN 47001(US)**

(74) Representative: **Canonici, Jean-Jacques et al**
**Procter & Gamble European Technical**
**Center N.V. Temselaan 100**
**B-1820 Strombeek-Bever(BE)**

(54) **Mineral supplements with sugar alcohols.**

(57) Sugar alcohols such as sorbitol are used to enhance mineral bioavailability from mineral-fortified compositions, especially orange juice. A method for preparing stable, iron/calcium-fortified juices incorporating sugar alcohols is provided. Mixed fortification with iron and calcium is secured.

EP 0 343 703 A2

## MINERAL SUPPLEMENTS WITH SUGAR ALCOHOLS

### TECHNICAL FIELD

The present invention relates to improvements in nutritious mineral supplements which contain iron compounds and calcium compounds. In particular, methods for preparing stable nutritious beverages having enhanced mineral bioavailability are provided.

### BACKGROUND OF THE INVENTION

Vitamin and mineral supplements for human and veterinary use are commonplace. Recently, it has become recognized that certain groups of the human population may require quite high intakes of minerals, such as calcium, to prevent or alleviate certain disease states, for example, osteoporotic conditions. The medical management of certain anemias can be handled rather well by increasing the daily intake of iron. Some diets, or heavy physical exercise, may require the intake of considerable quantities of minerals apart from those generally obtained through what otherwise would be considered a balanced diet.

Mineral supplements, such as those commercially available, are useful in many circumstances where enhanced mineral uptake is desirable. However, adhering to a regimen which requires the separate intake of mineral supplements can give sub-optimal results, simply because the regimen requires a change in the normal habits and practices of the user. It would be more convenient if the minerals could be administered conjointly, so that they would be ingested without extra attention, planning and implementation on the part of the user.

There are well-recognized problems associated with adding mineral supplements to foods and beverages. For example, many calcium supplements tend to be rather insoluble, and, therefore, not very useful in beverages, or tend to have a "chalky" taste or mouth feel. Iron supplements tend to discolor foodstuffs, or to be organoleptically unsuitable. Moreover, it is particularly difficult to formulate foods and, especially, beverages, containing mixtures of iron supplements and calcium supplements, inasmuch as these minerals tend to interact. This interaction not only affects the organoleptic and aesthetic properties of the foods and beverages, but also undesirably affects the nutritional bioavailability of these minerals, themselves.

It would be desirable, therefore, to have iron plus calcium supplements wherein bioavailability of both minerals is optimized. It would also be quite useful to have such supplements which can be used in food and beverage compositions without undesirably affecting organoleptic or aesthetic properties.

It is an object of the present invention to provide mixed iron-calcium mineral supplements which fulfill these unmet needs.

It is a further object of this invention to provide foodstuffs, beverages and beverage concentrates which are supplemented with iron and calcium.

It is a further object herein to provide means for enhancing biological uptake of iron and calcium minerals, especially from citrus beverages, particularly orange or grapefruit juice, in compositions which are both palatable and stable.

These and other objects are secured herein, as will be seen from the following disclosure.

### BACKGROUND ART

The use of sugar alcohols, including sorbitol, in calcium mineral supplements, including beverages, is disclosed in Japanese Laid-Open Patent Journal, SHO 59-31710, Feb. 20, 1984, Masaki Takahara, date of application Aug. 17, 1982. A composition containing orange juice is disclosed.

The use of D-sorbitol to enhance iron uptake in rats is described by Herndon, et al, J. Nutrition 64, 615 (1958).

The depression of iron absorption by high levels of calcium was recognized as early as 1940. Since then, various groups have repeatedly confirmed the significant inhibition of iron absorption by calcium. Adverse effects of calcium, including decrease in hemoglobin regeneration, reduced whole body iron retention and delayed restoration of tissue and blood iron levels have been reported. In postmenopausal women, calcium supplements, namely, calcium carbonate and calcium hydroxyapatite, markedly reduced iron absorption. In addition, calcium carbonate in prenatal multivitamin mineral supplements was identified as an inhibitor of absorption in nonpregnant women. Thus, individuals that consume high calcium and marginal amounts of iron simultaneously could develop iron deficiency anemia. See: Kletzein, S. W. (1940). Iron Metabolism. J. Nutr. 19, 187-97; Chapman, D. G. and Campbell, J. A. (1957). Effect of Calcium and Phosphorus Salts on the Utilization of Iron by Anemic Rats. Br. J. Nutr. 11, 127-133; Dunn, J. A. (1968). The Effects of Dietary Calcium Salts and Fat on Iron Absorption in the Rats. S. Afr. J. Med. Sci. 33, 65-70; Barton, J. C., Conrad, M. E. and Parmley, R. T. (1983). Calcium Inhibition of Inorganic Iron Absorption in Rats. Gastroenterology 84, 90-101; Dawson-Hughes, B., Seligson, F. H. and Hughes, V. A. (1986). Effects of Calcium Carbonate and Hydroxyapatite on Zinc and Iron Retention in Postmenopausal Women. Am. J. Clin. Nutr. 44, 83-88; and Seligman, P. A., Caskey, J. M., Frazier, J. L., Zucker, R. M., Podell, E. R. and Allen, R. M. (1983). Measurement of Iron Absorption from Prenatal Multivitamin Supplements. Obstetrics and Gyn. 61, 356-362.

For many decades orange juice has been recognized as an enhancer to iron absorption. Orange juice consumed with a typical western type breakfast reportedly increased iron bioavailability by 2.5-fold. In an in vitro system, addition of orange juice to breakfast meals and cooked pinto beans reportedly caused a dramatic increase in iron solubility. According to Rossander, et al, the reduction of iron absorption by tea was alleviated by orange juice. See: Lynch, S. R. and Cook, J. D. (1980), Interaction of Vitamin C and Iron. Annals New York Academy of Sciences, 32-44; Rossander, L., Hallberg, L. and Bjorn-Rasmussen, E. (1979), Absorption of Iron from Breakfast Meals. Am. J. Clin. Nutr. 32, 2484-2489; Carlson, B. L. and Miller, D. D. (1983), Effects of Product Formulation, Processing and Meal Composition on In Vitro Estimated Availability from Cereal Containing Breakfast Meals. J. Food Sci. 48, 1211-1216; and Kojima, N., Wallace, D. and Bates, W. G. (1981), The Effects of Chemical Agents, Beverages and Spinach on the In Vitro Solubilization of Iron from Cooked Pinto Beans. Am. J. Clin. Nutr. 34:1392-1401.

See, also, the nutritional literature: Ting, S. V. (1980). Nutrients and Nutrition of Citrus Fruits in "Citrus Nutrition and Quality" (edit. Nagy, S. and Attaway, J.). Amer. Chem. Soc., pp. 3-24; Gillooly, M., Bothwell, T. M., Torrace, J. D., MacPhail, A. P., Derman, D. P., Bezwoda, W. R., Mills, W. and Charlton, R. W. (1983). The Effects of Organic Acids, Phytates and Polyphenols on the Absorption of Iron from Vegetables. Br. J. Nutr. 49, 331-342; Hallberg, L. and Rossander, L. (1984). Improvement in Iron Nutrition in Developing Countries: Comparison of Adding Meat, Soy Protein, Ascorbic Acid, Citric Acid and Ferrous Sulfate on Iron Absorption for a Simple Latin American Type of Meal. Am. J. Clin. Nutr. 39; 577-583; and Kelly, S. E., Chawla-Singh, K., Sellin, J. M., Yasillo, N. J. and Rosenberg, I. M. (1984). Effects of Meal Composition on Calcium Absorption: Enhancing Effect of Carbohydrate Polymer. Gastroenterol. 87, 596-600.

In addition to the foregoing, various mineral supplements, including iron supplements and calcium supplements, are described in the following references.

Certain forms of calcium citrate-malate are disclosed for use as mineral supplements, including beverages; see Japanese Application Sho 54-173172, date of application December 28, 1979, laid-open Sho 56-97248, August 5, 1981; and see also French Patent 2,219,778 (Application 73.08643).

Some form of iron sucrate has been administered to children and the effect on Hb reported; see the Russian reference Metreveli, E.G., PEDIATRIYA (Moscow) 1977, (12), 17-19; C. Abs. 89:637.

Remington's Pharmaceutical Sciences, 15th Ed., 393 (1975) indicates that ferrous and ferric ions form soluble coordination complexes with many agents such as ammonium salts, citrates, tartrates, amines, sugar and glycerine, which protect the iron from precipitation by the usual iron precipitants. Iron gluconate and fumarate salts are said to be employed as hematinics.

Goodman and Gilman, The Pharmacological Basis of Therapeutics, 5th Ed., 1315-1316 (1975) reports that iron salts have many incompatibilities and should be prescribed alone, preferably between meals, for maximal absorption, but just after meals if necessary to minimize gastric symptoms. Gastrointestinal absorption of iron is reportedly adequate and essentially equal from the following six ferrous salts: sulfate, fumarate, gluconate, succinate, glutamate, and lactate. Absorption of iron is lower from ferrous citrate, tartrate, pyrophosphate, etc. Reducing agents such as ascorbic acid and some chelating agents such as succinic acid may increase absorption of iron from ferrous sulfates, but are said to be not worth the extra cost because of the high efficacy of ferrous sulfate when administered alone. Ferrous sulfate is reported to have a saline, astringent taste, and is mixed with glucose or lactose to protect it against oxidation, when used as an iron supplement.

European Patent 164,657 to Pfeiffer and Langden relates to an iron dextran, which is obtained by adding precipitated ferric hydroxide to dextran produced by adding sucrose solution to a solution of D-glucose and dextran-sucrose enzyme.

U.S. Patent 4,582,709, to Peters and Derick, April 15, 1986, relates to chewable mineral supplements, and lists, inter alia, various calcium and iron compounds.

U.S. Patent 4,351,735, to Buddemeyer, et al, September 28, 1982, relates to mineral supplements which contain certain phosphate moieties. Dispersibility of the compositions is said to be enhanced by "hydroxyl sources", e.g., sugars.

U.S. Patent 4,214,996, to Buddemeyer, et al, July 29, 1980, relates generally to the same subject matter as the '735 patent, above, but claims, inter alia, iron compositions and calcium compositions.

The beneficial effect of orange juice on the uptake of iron from dietary sources is described by Carlson and Miller in JOURNAL OF FOOD SCIENCE 48, 1211 (1983).

U.S. Patent 2,325,360, to Ayres et al, issued July 27, 1943, discloses a method for replacing gases removed during deaeration of fruit juices, such as orange juice, with carbon dioxide. In this method, dry calcium carbonate, or a mixture of calcium carbonate and citric acid, is dropped into a can which is then filled with deaerated orange juice. (Other organic acids such as malic and tartaric acid can be used in place of citric acid.)

U.S. Patent 3,657,424, to Akins et al, issued April 18, 1972, discloses the fortification of citrus juices, including orange juice, with sodium, calcium and chloride ions in amounts beyond what is naturally present in the juice. Calcium salts which can be used in fortification include the chlorides, citrates or phosphates, although calcium chloride is preferred for providing the desired chloride ion.

U.S. Patent 3,114,641, to Sperti et al, issued December 17, 1963, discloses extended orange juice products obtained by diluting single-strength orange juice or concentrated orange juice. To maintain the flavor of the diluted orange juice product, materials such as calcium chloride, magnesium chloride, sodium or potassium citrates, tartaric and malic acids (or their salts) are included.

British Patent Specification 2,095,530, published October 6, 1982, discloses a process for obtaining an acid beverage enriched in protein, particularly a fruit juice or fruit-flavored beverage. In this process, an aqueous suspension of soy protein is prepared using water and/or fruit juice. Calcium in a concentration of from 5 to 50mM is added, after which the pH of the suspension is reduced and the insoluble material separated to yield a protein solution. A fruit juice or fruit flavoring can then be added to this protein solution. The calcium can be added in the form of the chloride, acetate, tartrate, malate or lactate salt.

European Patent Application 75,114, published March 30, 1983, discloses protein-containing fruit juice drinks enriched with vitamins and minerals. These drinks contain 30-90% fruit juice (a mixture of 20-70% apple juice, 4-40% white grape juice, 1-10% passion fruit juice and 5-25% lemon juice), 2 to 20% whey protein concentrate, and a mineral salt mixture of potassium, sodium, magnesium, calcium and phosphate. Calcium is present in these drinks at 0.01 to 0.3%, preferably at 0.02 to 0.03%.

The recently-allowed United States Patent Application Serial No. 860,607, filed May 7, 1986, entitled FRUIT JUICE BEVERAGES AND JUICE CONCENTRATES NUTRITIONALLY SUPPLEMENTED WITH CALCIUM, D. C. Heckert, incorporated herein by reference, relates inter alia to orange juice fortified with calcium citrate malate, prepared and processed in a manner preferred in the practice of the instant invention.

## SUMMARY OF THE INVENTION

The present invention encompasses nutritional mineral supplements which comprise a mixture of:
  i) a nutritionally supplemental amount of an iron source;
  ii) an absorption-enhancing amount of a sugar alcohol; and
  iii) a nutritionally supplemental amount of a calcium source.

The mineral supplements herein most preferably employ iron-sugar complexes as the iron source, especially iron-sugar complexes of the type described more fully hereinafter whose counterions are selected from malate, citrate, tartrate, ascorbate, or mixtures thereof. Iron-sugar complexes such as iron sucrate-malate, iron fructate-malate, or mixtures thereof, are especially preferred, and are most preferred when the iron is in the ferrous (+ II) state, although ferric (+ III) iron is also acceptable.

Various sugar alcohols can be used herein, and sorbitol, mannitol and mixtures thereof, are convenient. Sorbitol is preferred.

As is disclosed hereinafter, various calcium sources can be used in the practice of this invention, but calcium citrate-malate is preferred.

This invention also encompasses foods, beverages or beverage concentrate compositions comprising:
  a) a foodstuff, beverage or beverage concentrate;

b) a nutritionally supplemental amount of a calcium supplement;

c) a nutritionally supplemental amount of an iron-sugar complex; and

d) an absorption-enhancing amount of a sugar alcohol.

In such compositions, the calcium supplement is preferably calcium citrate-malate, and the iron-sugar complex is preferably selected from iron sucrate-malate, iron fructate-malate, iron sucrate-citrate, iron fructate-citrate, iron sucrate-ascorbate, iron fructate-ascorbate, or mixtures thereof. (The iron is preferably in the ferrous state.)

Again, various sugar alcohols can be used in such compositions, with sorbitol, especially D-sorbitol, being preferred.

Typical beverage or beverage concentrate compositions according to this invention comprise:

a) at least about 0.1% by weight of fruit or cola flavor, or at least 3% by weight of fruit juice;

b) a nutritionally supplemental amount of calcium citrate-malate;

c) a nutritionally supplemental amount of iron II sucrate-malate; and

d) at least about 1% by weight of sorbitol.

By way of example, the fruit juices used herein include grape juice, pear juice, passion fruit juice, pineapple juice, banana juice or banana puree, apricot juice, orange juice, lemon juice, grapefruit juice, apple juice, cranberry juice, tomato juice, and mixtures thereof.

The invention encompasses beverages, especially juice and cola beverages, which are carbonated in the manner of soft drinks, as well as "still" beverages. The invention also encompasses nectars and full-strength beverages or beverage concentrates which contain at least about 45% by weight of juice.

The nutritional supplements herein are particularly useful with beverages or beverage concentrates made from orange juice or grapefruit juice.

As will be disclosed more fully hereinafter, the mineral supplements of this invention also can conveniently be used in powder, tablet, chewable lozenge, capsule or liquid form, for enteral or parenteral nutrition, and in combination with conventional foodstuffs, such as breads, cakes, snacks, infant formulations, meat analogs and extenders, spreads, and the like.

All ratios, proportions and percentages herein are by weight, unless otherwise specified.

## DETAILED DESCRIPTION OF THE INVENTION

The present invention involves the use of sugar alcohols to enhance the body's uptake of nutritionally-supplemental amounts of iron compounds, or mixtures of iron and calcium compounds, in humans and lower animals.

By "nutritional" or "nutritionally-supplemental amount" herein is meant that the mineral sources used in the practice of this invention provide a nourishing amount of said minerals. In mineral supplements such as tablets or powders, this supplemental amount will comprise at least 3% of the Recommended Daily Allowance (RDA) of the daily intake of said mineral, as defined in The United States of America (see Recommended Daily Dietary Allowance-Food and Nutrition Board, National Academy of Sciences-National Research Council). More generally, mineral supplements will contain at least 10%, more typically 50% to 300%, of the RDA per unit dose of the supplement. In food or beverage products of the type disclosed herein, the nutritionally supplemental amount will generally comprise more than 3% of the RDA, preferably 10%-100% RDA, most preferably 10%-30% of the RDA, per unit portion of the food or beverage product. Of course, it is recognized that the preferred daily intake of any mineral may vary with the user. For example, pregnant, lactating, or post-menopausal females may require an increased intake of calcium, over the usual RDA. Persons suffering with anemia may require an increased intake of iron. Such matters are familiar to physicians and nutritional experts, and usage of the compositions of the present invention may be adjusted accordingly.

In general, the RDA (iron) ranges from 10 mg per 6 Kg to 18 mg per 54-58 Kg female, depending somewhat on age. As is well-known, it is possible to overdose with iron supplements, especially in males, with deleterious effects to the liver. Typically, foods and beverages are supplemented with only about 10-15% RDA iron (based per serving) to account for iron which is available from other dietary sources (assuming a reasonably balanced diet), thereby avoiding this problem. In general, the RDA (calcium) will range from 360 mg per 6 Kg for infants to 1200 mg/54-58 Kg female, depending somewhat on age. Moreover, it can be difficult to supplement beverages with more than 20-30% RDA of calcium (based per serving) without encountering precipitation and/or organoleptic problems. However, this level of supplementation is equivalent to cow's milk in calcium value, and is quite acceptable. Of course, if iron toxicity and organoleptic quality are not deemed important considerations in individual circumstances, more of the supplements herein can be used.

The "iron-sugar" complexes preferred for use in the practice of this invention are prepared in the manner described more fully hereinafter. (These materials are referred to herein as "complexes", but they may, in fact, exist in solution as complicated, highly-hydrated, protected colloids. However, the term "complex" is used herein for simplicity.) While the iron in these complexes can be in the ferric (iron III) state, it is more preferably in the ferrous (iron II) state. Ferrous iron is better tolerated and utilized by the body than ferric iron. Importantly, ferric iron and common ferrous salts can cause off-flavors in some beverages, after storage; ferric iron can also oxidize and thus degrade ascorbic acid (Vitamin C) in citrus beverages. The preferred complexes used herein can conveniently be thought of as iron-sugar-carboxylate complexes, wherein the carboxylate provides the counterion for the ferrous (preferred) or ferric iron. While not intending to be limited by theory, it is believed that the acceptable taste of these iron complexes is due to the relatively large sizes of the sugar moiety and carboxylate counterion, which mask the usual "well-water" and/or brackish flavor of some iron supplements.

The overall synthesis of the preferred iron-sugar-carboxylate complexes used in the practice of this invention involves:

a) forming a calcium-sugar moiety in aqueous media, for example, by reacting calcium hydroxide with a sugar;

b) reacting an iron source, such as ferrous ammonium sulfate, with the calcium-sugar moiety in aqueous media to provide an iron-sugar moiety; and

c) neutralizing the reaction system with a carboxylic acid, for example, malic acid, to provide the desired iron-sugar complex.

The preferred iron II-sucrate-malate complex prepared in this manner is essentially equivalent to ferrous sulfate in iron bioavailability (measured as % change in hematocrit of test animals over the range of 0-9 ppm Fe), and, most importantly, is organoleptically acceptable in beverages, especially citrus beverages.

The "sugars" which can be employed in the preparation of iron compounds preferred for use in the practice of this invention include any of the ingestible saccharidic materials, and mixtures thereof, well-known in the culinary arts. For example, glucose, sucrose and fructose can conveniently be employed, with sucrose and fructose being the more preferred. However, other saccharidic materials can be used, for example mannose, galactose, lactose, maltose, and the like.

The "carboxylate counterion" used in the preparation of the preferred iron-sugar complexes herein can be any ingestible carboxylate species. However, some judgment must be made with regard to flavor contribution. For example, citrate, malate and ascorbate yield ingestible complexes whose flavors are judged to be quite acceptable, particularly in fruit juice beverages. Tartaric acid is acceptable, particularly in grape juice beverages, as is lactic acid. Longer-chain fatty acids may be used in solid mineral supplements, but can affect flavor and water solubility. For essentially all purposes, the malate (preferred), citrate and ascorbate moieties suffice, although others can be selected, according to the desires of the formulator.

In a less preferred mode, the counterion for the iron-sugar complex can be noncarboxylate moieties such as phosphate, chloride, sulfate, or the like. However, such counterions can undesirably interact with calcium ions, especially in beverages. In high concentrations, these counterions may contribute an undesirable flavor note. Accordingly, the carboxylate counterions noted above are preferred herein.

The preparation of the preferred calcium source used herein, "calcium citrate-malate", is described hereinafter in considerable detail.

The "sugar alcohols" used in the practice of the present invention are well-known materials, many of which are widely distributed in nature, or which can be prepared by the reduction of sugars. Included among such sugar alcohols are materials such as sorbitol (preferred for use herein), mannitol, galactitol, and the like. Mixtures of sugar alcohols, such as the mixture of mannitol and sorbitol which is prepared by the electrolytic reduction of glucose at pH 10-13, can also be employed herein.

The sugar alcohols herein are used in an "absorption-enhancing" amount. By "absorption-enhancing" is meant that the sugar alcohols enhance the bioavailability of the iron/calcium present in the compositions. As noted above, iron bioavailability is normally somewhat impaired by the conjoint administration of calcium, but this impairment is overcome by use of the sugar alcohols in an absorption-enhancing amount in the manner of this invention. In general, compositions of the present invention will contain at least about 1% by weight of the sugar alcohol as the absorption-enhancing amount. Typically, the sugar alcohol will be used at levels of 1%-4% in the present compositions and processes, but considerably higher amounts (generally, up to 30% in liquid formulations and 75%-85% in solids such as tablets, capsules, lozenges, and the like) can be used according to the desires of the formulator. As is known, the sugar alcohols do display some sweetness qualities, and this can be taken advantage of by the formulator. For beverages such as orange juice or grapefruit juice where it would be undesirable to have excessive sweetness, usage ranges of from about 1.5% to about 2.5%, most preferably about 2%, by weight of sorbitol serve not only to provide enhanced absorption of iron/calcium mixtures, but also to "smooth" the flavor impression of the juice products without introducing an undesirable sweetness. Moreover, usage of sugar alcohols (especially sorbitol) at these levels in grapefruit juice moderates the bitter note sometimes detected therein.

The following illustrates the preparation of some preferred iron compounds for use in the practice of this invention, but is not intended to be limiting thereof.

Preparation of Iron II Sucrate-Malate

Sucrose (85.5 g) is dissolved in water (299.8 g), making sure that dissolution is complete. Calcium hydroxide (18.5 g) is then added, and the mixture is stirred for 5 minutes. Any clouding is observed, and the resulting solution is filtered through a glass filter.

To the resulting calcium-sucrate solution is added ferrous ammonium sulfate (24.5 g), and the solution is covered air-tight (e.g., SARAN WRAP). The green color indicates the iron is in the desired II oxidation state.

To the above solution is added malic acid (33.5 g) in 3 batches, to pH 3-4. The precipitate is filtered through standard filter paper, but the filter cake comprising calcium sulfate is not rinsed. The resulting solution comprises the iron sucrate-malate used in the practice of this invention. The solution can be used per se, or can be freeze-dried to provide the iron sucrate-malate in powder form.

In an alternate mode, KOH can be substituted for Ca(OH)$_2$ in the first step, but sulfate ion will be left in the final product.

Variations in the method for preparing iron-sugar complexes, as well as alternate sugars and counterions, are given in the following examples.

Iron II Fructate Malate

Fructose (360 g; 2 moles) is dissolved in water (1644 g), making sure all fructose is dissolved. Calcium hydroxide (148 g; 2 moles) is added to the fructose solution and stirred for 5 minutes. The solution is filtered through a glass filter.

To the calcium fructose solution is added iron II ammonium sulfate (196 g; 0.5 mole) and the solution is covered air-tight with SARAN WRAP. The color should remain green. Malic acid (268 g; 2 moles) is added in three batches. At each addition, a pH reading is taken with litmus paper to insure pH 3-4. The precipitate is filtered off (paper filter). The title compound is in the filter liquor.

Iron II Sucrate-Citrate

Sucrose (684 g; 2 moles) is dissolved in water 2399 g), making sure all sugar is dissolved. Calcium hydroxide (148 g; 2 moles) is added to the solution and stirred for five minutes. The solution is filtered through a glass filter. To the calcium-sucrate solution is added iron II ammonium sulfate (196 g; 0.5 mole) and the solution is covered air-tight with SARAN WRAP. The green color should persist. Citric acid (384 g; 2 moles) is added to the reaction mixture in three batches. At each point of addition, a pH reading is taken with litmus paper to insure pH 3-4. The precipitate is filtered-off (paper filter) and the filter cake is not rinsed. The title compound is in the filter liquor.

Iron II Sucrate-Tartrate

Sucrose (684 g; 2 moles) is dissolved in water (2399 g), making sure all sugar is dissolved. Calcium hydroxide (148 g; 2 moles) is added to the sugar solution and stirred for 5 minutes. The solution is filtered through a glass filter.

To the calcium-sucrate solution is added iron II ammonium sulfate (196 g; 0.5 mole) and the solution is covered air-tight with SARAN WRAP. The green color should persist. Tartaric acid (300 g; 2 moles) is added to the solution in three batches. At each time of addition, a pH reading is taken with litmus paper to insure pH 3-4. The precipitate is filtered (paper filter) and removed; the filter cake is not rinsed. The title compound is in the filter liquor.

Iron II Glucate/Fructate-Malate

Glucose (360 g; 2 moles) and fructose (360 g; 2 moles) are co-dissolved in water (1643 g), making sure all sugar is dissolved. Calcium hydroxide (148 g; 2 moles) is added to the sugar-water and stirred for 5 minutes. The solution is filtered through a glass filter.

To the calcium/mixed sugars solution is added iron II ammonium sulfate (196 g; 0.5 moles) and the solution is covered air-tight with SARAN WRAP. The green color should persist. Malic acid (268 g; 2 moles) is added in three batches. At each addition, a pH reading is taken with litmus to insure pH 3-4. The precipitate is filtered-off (paper filter) and the filter cake is not rinsed. The title compound is in the filter liquor.

Iron II Sucrate-Citrate/Ascorbate

Sucrose (684 g; 2 moles) is dissolved in water (2399 g), making sure all sugar is dissolved. Calcium hydroxide (148 g; 2 moles) is added to the sugar water solution and stirred for 5 minutes. The solution is filtered through a glass filter.

To the calcium-sucrate solution is added iron II ammonium sulfate (196 g; 0.5 mole) and the solution is covered air-tight with SARAN WRAP. The green color should persist. The citric acid (192 g; 1 mole) is first added to the solution, then the ascorbic acid (352 g; 2 moles) is added in three batches. At each time of addition, a pH reading is taken with litmus paper to insure pH 3-4. The precipitate is filtered (paper filter). The title compound is in the filter liquor.

The preparation of a preferred calcium source for use in the present compositions is as follows.

Preparation of Calcium Citrate-Malate

A calcium citrate-malate solution is prepared by dissolving 2 parts sucrose and then 0.1 part citric and 0.28 part malic acids in 28.19 parts water. Calcium hydroxide (0.22 part) is added and the mixture is agitated. This solution can be used directly to prepare beverages, or can be freeze-dried to use in solid mineral supplements.

Citrus Products

The present invention is particularly suited for the preparation of juice beverages and beverage concentrates, particularly orange juice. The concentrated orange juice, orange juice aroma and flavor volatiles, pulp and peel oils used in the method of the present invention can be obtained from standard orange juice processing. See Nagy et al, Citrus Science and Technology, Volume 2, (AVI Publishing Co. 1977), pp 177-252 (herein incorporated by reference) for standard processing of oranges, grapefruit and tangerines. (See also Nelson et al, Fruit and Vegetable Juice Processing Technology (3rd Ed., AVI Publishing 1980),pp. 180-505 (herein incorporated by reference) for standard processing of noncitrus juices such as apple juice, grape juice, pineapple juice, etc. to provide sources of juice and juice materials for mineral-supplemented noncitrus juice products). Fresh juice is extracted from the oranges, principally of the Valencia type. (The peel of the oranges is initially rasped to provide peel oils which can be used in the method of the present invention.) Juices from different oranges are frequently blended to adjust the sugar to acid ratio. A sugar to acid ratio of from about 8:1 to about 20:1 is considered acceptable. However, preferred sugar to acid ratios are typically from about 11:1 to about 15:1.

Juice is extracted from the oranges by using automatic juicing machines, or less often by hand squeezing of the oranges. The type of equipment used to extract the juice is not critical. The raw juice exiting from the squeezing device contains pulp, rag and seeds. The rag and seed are separated from the juice and pulp in a finisher. The juice is then typically separated into a pulp portion and a serum portion. (The pulp portion can be used as a source of pulp in the method of the present invention.)

The serum portion can be concentrated by a variety of techniques which typically include evaporative concentration or freeze concentration. In evaporative concentration, the serum portion of the juice is passed through an evaporator (e.g., falling film or temperature accelerated short time evaporator [TASTE] type). Water vapor, as well as the aroma and flavor volatiles, are stripped from the juice. These stripped volatiles are then centrifuged to provide an upper layer (essence oils) and a lower layer (aqueous essence). (A portion of these essence oils and aqueous essence are typically used as the source of orange juice aroma and flavor volatiles for the method of the present invention.) The remaining stripped juice is then concentrated in the evaporator (by heat) to the appropriate amount of solids as measured by the sugar content of the concentrated juice. This concentrated juice can then be used in the method of present invention.

Most concentrated orange juices are obtained by evaporative concentration. However, freeze concentration can also be used to obtain concentrated orange juice useful in the method of the present invention. Freeze concentration typically involves passing the serum portion of the juice through a scraped wall heat exchanger to form substantially pure ice crystals which are then separated from the concentrated juice. A preferred freeze concentration method is disclosed in U.S. Patent 4,374,865 to Strobel, issued February 22, 1983, which is incorporated by reference. Unlike evaporative concentration, concentrated orange juice obtained by freeze concentration typically contains the aroma and flavor volatiles as well.

## Method for Preparing Beverages and Beverage Concentrates Supplemented with Iron/Calcium

The preferred overall method for preparing the liquid compositions herein involves preparing premix solutions of the iron and calcium complexes (see above) and admixing the premixes to the liquid compositions. The sugar alcohol is added in the desired amount, either as a solid or as a concentrated solution (e.g., 70% aqueous sorbitol). The following discussion of this method will generally be with regard to formation of orange juice beverages (including diluted 10-60% diluted "nectars") and juice concentrates, which are highly preferred fruit juice products according to the present invention. However, this method can also be used to prepare iron- and calcium-supplemented beverages and concentrates, especially those based on other citrus juices such as grapefruit juice, noncitrus juices such as apple juice, as well as mixtures of juices.

Since juice compositions comprising the sugar alcohol and both the iron and the calcium supplements are more complicated to prepare than compositions containing only the sugar alcohol and iron, preparation of the former compositions is described in detail.

In general, an acid component comprising citric acid and malic acid is typically dissolved in the appropriate quantity of water. (If desired, fruit juice or concentrated fruit juice such as lemon juice can be used to supply a portion of the acids.) Generally, this acid component comprises from 0 to about 90% by weight citric acid and from about 10 to 100% by weight malic acid. For orange juice, this acid component typically comprises from about 20 to about 90% by weight citric acid and from about 10 to about 80% by weight malic acid. Preferably, this acid component comprises from about 5 to about 60% by weight citric acid and from about 40 to about 95% by weight malic acid. (For noncitrus juices such as apple juice, this acid component typically comprises from about 5 to about 80% by weight citric acid and from about 20 to about 95% by weight malic acid, and preferably comprises from about 20 to about 50% by weight citric acid and from about 50 to about 80% by weight malic acid.) As a rule, the ratio of these acids is selected to provide optimum flavor character in the juice.

Once the solution containing the dissolved acids is formed, a source of calcium is then added. Calcium carbonate ($CaCO_3$) is a preferred calcium source. This calcium source leads to the greatest and most rapid initial solubilization of calcium and causes the least amount of off-flavor generation. Calcium hydroxide [Ca(OH)$_2$] and calcium oxide (CaO) are also acceptable calcium sources, but can cause more off-flavor generation than calcium carbonate. The weight ratio of total acids to calcium added in the solution is typically from about 0.5 to about 12. Preferably, this weight ratio is from about 1 to about 6.

Addition of calcium carbonate, calcium oxide, or calcium hydroxide to the aqueous solution of acids provides a premix containing soluble and solubilizable calcium. This is due to the fact that highly soluble calcium citrate and malate species such as CaHcitrate, Ca(H$_2$citrate)$_2$, and CaHmalate are formed in the solution due to the reaction between the calcium source and the acids. Without added stabilizers, the highly soluble calcium citrate species are stable in the premix solution for periods up to only about a few hours.

After this short period of time, the highly soluble citrate species tend to disproportionate to the corresponding acid and the more thermodynamically stable, insoluble calcium citrate salts, such as $Ca_3$ citrate$_2$.

To improve the stability of the more soluble calcium malate and especially citrate species in the premix solution, it is preferred in the method of the present invention to include a premix stabilizer. Materials which can complex with calcium and/or act as crystallization inhibitors are useful as premix stabilizers. These materials include sugars, such as sucrose, glucose, fructose, high fructose corn syrup, invert sugar, and polysaccharides such as pectin, algins, hydrolyzed starches, xanthan gum, and other edible gums. Concentrated juices which naturally contain both sugars and polysaccharides are particularly suitable premix stabilizers. Preferred premix stabilizers are sucrose and high fructose corn syrup (especially for extended juice products) and concentrated orange juice having a sugar content of from about 35 to about 80° Brix whose source is described hereafter.

The premix stabilizer can be added immediately after the calcium source is added to the aqueous solution containing the acids. (When calcium carbonate is the calcium source, carbon dioxide evolution is preferably allowed to substantially cease before the premix stabilizer is added.) However, if desired, the premix stabilizer (especially in the case of sugars and concentrated juice) can be added to the aqueous solution of the acids prior to addition of the calcium source. The amount of premix stabilizer included in the premix solution typically depends upon the stabilizer used. When sugars are used as the premix stabilizer, they are typically added in an amount sufficient to provide a sugar content of from about 2 to about 40° Brix. When polysaccharides are used, the amount can vary widely, but is typically from about 0.01 to about 0.5% on a weight/volume basis. When concentrated juice is used as the premix stabilizer, it is typically included in an amount sufficient to provide a sugar content of from about 2 to about 40° Brix (preferably from about 2 to about 24° Brix).

The premix solution of solubilized and solubilizable calcium is typically prepared in a batch-type fashion, as in the description above, at room temperature. However, this premix solution can also be prepared in a continuous fashion. In this continuous method, the ingredients (water, acids, calcium source and optional premix stabilizer) are constantly metered together to form the premix solution. The level at which the ingredients are metered is adjusted, as necessary, to insure appropriate solubilization of the calcium in the premix solution and to provide the appropriate acidity.

Separately, a premix solution of the iron-sugar complex is prepared. In general, this solution is somewhat simpler to prepare than the calcium citrate-malate solution, above, since precipitation is not a major problem. Thus, a calcium-sugar reaction product is treated with an iron (preferably iron II) source, and the reaction product is neutralized with a carboxylic acid, in the manner described hereinabove.

The premix solution containing the solubilized calcium and the premix containing the solubilized iron are combined in a mix tank with chilled (e.g., below about 4.4° C) concentrated orange juice having a sugar content of from about 35 to about 80° Brix (preferably from about 60 to about 70° Brix), orange juice aroma and flavor volatiles, plus other orange juice materials such as pulp and peel oils, to provide iron- and calcium-supplemented orange juice products. The particular proportions of premix solution, concentrated juice, aroma and flavor volatiles, pulp and peel oils used will depend upon a number of different factors, including the type of orange juice product involved (single-strength juice beverage or juice concentrate). For example, iron- and calcium-supplemented 42° Brix orange juice concentrates can be prepared by combining 65 parts concentrated orange juice (65° Brix), 5 parts pulp, 15 parts of an aroma/ flavor concentrate, 0.4 parts peel oil with the 15 parts Fe/Ca premix. Similar single-strength juice beverages can be prepared by appropriate variation of the amounts of concentrated orange juice, pulp, aroma/flavor concentrate, peel oil and premix solutions, as well as the inclusion of water.

Juice compositions and other beverages are preferably formulated at a pH below about 4.3, generally about 3.7-4.0, for reasons of microbial stability.

After the iron- and/or iron/calcium-supplemented orange juice product is obtained, it is then filled into cans, cartons, bottles or other appropriate packaging. In the case of orange juice concentrates, these products are typically frozen after being filled into cans.

The following example illustrates beverage compositions of the type provided by the practice of this invention, but is not intended to be limiting thereof.

<div align="center">EXAMPLE I</div>

Beverage Compositions

The following beverage compositions (a-i) are fortified with the iron (II) sucrate-malate and calcium citrate-malate prepared above to provide 10% RDA of iron and 100% RDA calcium per 180 ml serving. Sorbitol (2% wt. of product) is added to each composition to enhance iron/calcium bioavailability.

a) "sparkling" orange juice comprising 55% orange juice and 45% carbonated water;

b) pear-grapefruit nectar comprising 25% pear juice, 20% grapefruit juice, the balance comprising 10% sucrose-water;

c) kiwi-grapefruit drink comprising 20% kiwi fruit juice, 15% grapefruit juice, the balance comprising water;

d) mixed fruit "cocktail" comprising 10% each of the juices of passion fruit, mango, guava, pineapple, papaya, banana, apricot, mandarin orange, pear and lime juices;

e) yogurt/fruit beverage comprising 20% milk products, 1% pectin, 20% pineapple juice, 10% shredded pineapple fruit pulp, 16% corn syrup, the balance comprising water;

f) cola beverage comprising 0.35% cola flavor emulsion, 11% sugar, 0.1% phosphoric acid, 0.1% citric and malic acids, caramel coloring, the balance comprising carbonated water;

g) full-strength orange juice;

h) full-strength apple juice;

i) full-strength cow's milk.

The following example illustrates food compositions prepared in the manner of this invention, but is not intended to be limiting thereof.

## EXAMPLE II

## Food Compositions

The following food compositions (a-e) are fortified with the iron (II) sucrate-malate and calcium citrate-malate prepared above to provide 20% RDA of iron and 100% RDA calcium per 250 g serving. Sorbitol (2% wt. of product) is added to each composition to enhance iron bioavailability.

a) peanut butter product comprising finely ground peanuts, up to 3% peanut oil, salt;

b) cookie product comprising inner core of flour, shortening, flavoring and fructose enrobed in outer layer of flour, shortening, flavoring and sucrose;

c) brownie snack product comprising commercial DUNCAN HINES brownie mix;

d) soy-based meat analog product comprising a 50:1 (wt.) mixture of de-oiled soybean meal and egg whites, extruded, in patty or chunk form;

e) infant formulation in powder or liquid form comprising sterilized soy powder or soy "milk", vanilla flavor, preservative.

The following examples illustrate typical compositions of the present invention comprising both iron and calcium minerals.

## EXAMPLE III

Iron- and calcium-fortified chewable lozenges comprise:

| Ingredient | Amount |
|---|---|
| Iron II sucrate-malate | 20 mg |
| Calcium citrate-malate | 500 mg |
| Sodium citrate | 10 mg |
| D-sorbitol/fructose mix (1:1 wt.) | 5 g |
| Sodium ascorbate | 7 mg |
| Fruit flavor* | 6 mg |
| Color | As desired |

*Fruit flavors used herein generally comprise synthetically reconstituted flavor esters. In this example, pineapple flavor is used, and comprises a synthetic mixture of ethyl acetate, acetaldehyde, methyl n-valerate, methyl i-valer te, methyl i-caproate and methyl caprylate.

The lozenge of Example III is prepared by mixing the ingredients and compacting the mixture in a standard press.

EXAMPLE IV

Iron- and calcium-fortified orange juice comprises:

| Ingredient | Amount |
|---|---|
| Iron II sucrate-malate | 15 mg |
| Calcium citrate-malate | 300 mg |
| Citric acid | 20 mg |
| Sodium ascorbate | 10 mg |
| Sorbitol | 3.75 g |
| Orange juice | 150 g |

The calcium citrate-malate (aqueous solution form) and iron II sucrate-malate (aqueous solution form) are prepared. The sodium citrate and sodium ascorbate are dissolved in the orange juice, to which is then added the iron and calcium solutions.

EXAMPLE V

The composition of Example IV is modified by replacing the orange juice with apple juice or grapefruit juice, respectively, all other ingredients remaining the same.

EXAMPLE VI

A fortified beverage suitable for oral administration, including administration under stress conditions such as illness, eavy physical exertion or pregnancy, comprises:

EP 0 343 703 A2

| Ingredient | Amount |
|---|---|
| Mannitol | 30 g |
| Soy hydrolysate | 25 g |
| Iron II fructose-ascorbate | 50 mg |
| Calcium citrate-malate | 1500 mg |
| Sodium citrate | 250 mg |
| Sodium ascorbate | 250 mg |
| Multivitamin supplement* | 300 mg |
| Aqueous 30% dextrose | 250 g |
| Flavor | As desired |

*Comprising 100% RDA of Vitamins D, E, A. Vitamin K may be included if blood clotting is a consideration.

The mineral supplements herein can be provided using common inorganic sources of iron and calcium, as follows.

EXAMPLE VII

A mineral supplement powder in unit dose form is as follows:

| Ingredient | Amount |
|---|---|
| Ferrous fumarate | 35 mg |
| Sodium tartrate | 25 mg |
| Calcium carbonate | 1000 mg |
| Galactitol | 500 mg |

The powder is provided in a soluble gelatin capsule for oral ingestion.

## Claims

1. A nutritional mineral supplement, comprising a mixture of:
   i) a nutritionally supplemental amount of an iron source;
   ii) an absorption-enhancing amount of a sugar alcohol; and
   iii) a nutritionally supplemental amount of a calcium source.

2. A mineral supplement according to Claim 1 wherein the iron source is an iron-sugar complex, and wherein the counterion of the iron-sugar complex is selected from malate, citrate, tartrate, ascorbate, or mixtures thereof.

3. A mineral supplement according to Claim 2 wherein the iron-sugar complex is iron sucrate-malate, iron fructate-malate, or mixtures thereof.

4. A mineral supplement according to Claim 4 wherein the sugar alcohol is selected from sorbitol, mannitol and mixtures thereof.

5. A mineral supplement according to Claim 6 wherein the iron is in the ferrous state and which preferably contains a nutritionally supplemental amount of calcium citrate-malate.

6. A mineral supplement according to Claim 1, 2 or 5 wherein the sugar alcohol is sorbitol.

7. A food, beverage or beverage concentrate composition, comprising:
   a) a foodstuff, beverage or beverage concentrate; and
   b) a nutritional mineral supplement according to claims 1, 2, 3, 4, 5 or 6.

8. A beverage or beverage concentrate composition according to Claim 9, which comprises:
   a) at least about 0.1% by weight of fruit or cola flavor, or at least 3% by weight of fruit juice;
   b) a nutritionally supplemental amount of calcium citrate-malate;

13

c) a nutritionally supplemental amount of iron II sucrate- malate; and

d) at least about 1% by weight of sorbitol.

9. A composition according to Claim 13 wherein the fruit juice is selected from grape juice, pear juice, passion fruit juice, pineapple juice, banana juice or banana puree, apricot juice, orange juice, lemon juice, grapefruit juice, apple juice, cranberry juice, tomato juice, and mixtures thereof.

10. A juice beverage according to Claim 14 which is carbonated.

14